# EUROPEAN PATENT SPECIFICATION

(11) **EP 3 897 587 B1**
(45) Date of publication and mention of the grant of the patent: **15.04.2026**
(21) Application number: 19829588.3
(22) Date of filing: 20.12.2019
(51) Int. Cl.: A61K 9/28, A61K 9/50, A61K 47/38, A61K 31/519, A61P 35/00

(54) **PHARMACEUTICAL COMPOSITION COMPRISING IBRUTINIB**
PHARMAZEUTISCHE ZUSAMMENSETZUNG MIT IBRUTINIB
COMPOSITION PHARMACEUTIQUE COMPRENANT DE L'IBRUTINIB

(30) Priority: 21.12.2018 EP 18215288
(43) Date of publication of application: 27.10.2021
(73) Proprietor: Synthon B.V., 6545 CM Nijmegen (NL)
(72) Inventor: NOGUEIRAS NIETO, Luis, 08830 Sant Boi de Llobregat (ES); FRADERA GELABERT, Sara, 08830 Sant Boi de Llobregat (ES); ALVAREZ FERNANDEZ, Lisardo, 08830 Sant Boi de Llobregat (ES); KUMAR, Rohit, 08830 Sant Boi de Llobregat (ES); VIVANCOS MARTINEZ, Marta, 08830 Sant Boi de Llobregat (ES)
(74) Representative: Marciniak, Agata Maria
(86) International application number: PCT/EP2019/086585
(87) International publication number: WO 2020/127912

(56) References cited:
- WO-A1-2013/184572
- WO-A1-2014/004707
- WO-A1-2016/079216
- WO-A1-2017/174044
- WO-A2-2015/145415
- VÍT ZVONÍCEK ET AL: "Ibrutinib Polymorphs: Crystallographic Study", CRYSTAL GROWTH & DESIGN., vol. 18, no. 3, 7 March 2018 (2018-03-07), US, pages 1315 - 1326, XP055523340, ISSN: 1528-7483, DOI: 10.1021/acs.cgd.7b00923

## Description

### BACKGROUND OF THE PRESENT INVENTION

Ibrutinib, chemically 1-[(3*R*)-3-[4-amino-3-(4-phenoxyphenyl)-1*H*-Pyrazolo[3,4- *d*]pyrimidin-1-yl]piperidin-l-yl]prop-2-en-1-one of formula (I), 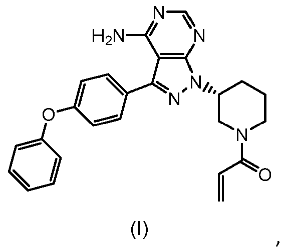 is a pharmaceutically active compound. It is used for the treatment of chronic lymphocytic leukaemia (CLL) in previously untreated patients and in patients who have received at least one previous treatment. It is also used for the treatment of mantle cell lymphoma (MCL) in patients whose disease does not respond to or has come back after previous treatment and to treat Waldenström's macroglobulinaemia (WM), also known as lymphoplasmacytic lymphoma, in patients who have had previous treatment or who cannot have chemo immunotherapy.

Ibrutinib is marketed by Janssen/Pharmacyclics under the brand name Imbruvica^{®} and is disclosed in WO2008039218. Imbruvica^{®} was initially only supplied as hard capsule in one strength: 140 mg. Patients take either 3 or 4 capsules once daily, depending on the disease to be treated. Recently, the FDA approved a new tablet formulation of Imbruvica^{®}. The tablet is available in the strengths 140, 280, 420 and 560 mg and thus reduces pill burden for patients.

Several crystalline forms of ibrutinib are described in literature. WO2013184572 discloses crystalline forms A, B and C and solvated forms D (methyl isobutyl ketone solvate), E (toluene solvate) and F (methanol solvate). The marketed product Imbruvica^{®}, both capsule and tablet formulation, contains crystalline ibrutinib form A. Other forms of ibrutinib are disclosed in CN103923084, WO2015081180, WO2015145415, WO2016025720, WO2016079216, CN105294696, CN105646484, CN105646499, CN105646498, WO2016160598, WO2016139588, WO2017029586, CN106117214, WO2017085628, CN107286163, EP3243824 and WO2018000250. Some of the described forms do contain unwanted solvents. Moreover, it was experienced in our laboratory that some of these forms are rather unstable.

Ibrutinib is a BCS class II compound, exhibiting low solubility and high permeability.

Both capsule and tablet composition of Imbruvica^{®} contain a considerable amount of the anionic surfactant sodium lauryl sulphate (SLS). It is known that this compound may give rise to irritation of the gastrointestinal tract.

It would be desirable to have a tablet composition comprising ibrutinib that exhibits adequate dissolution and is free of surfactant. The composition should be stable, suitable for production on commercial scale and bioequivalent to Imbruvica^{®}.

### BRIEF DESCRIPTION OF THE PRESENT INVENTION

The present invention provides a tablet composition comprising ibrutinib and one or more pharmaceutically acceptable excipients, characterized in that:
* The one or more pharmaceutically acceptable excipients are 10 to 30% w/w of one or more diluents, 0 to 7% w/w of one or more binders, 2 to 15% w/w of one or more disintegrants, 0.25 to 1.0% w/w of one or more glidants and 0.25 to 2.0% w/w of one or more lubricants; and
- Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation; and
- Ibrutinib form C is present in an amount from 60 to 80% w/w relative to the total weight of the tablet; and
- The composition is free of amiphiphilic molecules that contain both hydrophilic and lipophilic groups and have HLB (hydrophile-lipophile balance) number above 10; and
- The composition exhibits a dissolution rate of at least 65% in 20 minutes when tested in 900 ml 0.01 N hydrochloric acid pH 2.0 (+ 1 % Tween 20) at 37°C, 75 rpm and/or in 900 ml phosphate buffer pH 6.8 (+3% Tween 20) at 37°C, 75 rpm in a USP apparatus II.

It also provides a process for preparing the tablet composition comprising a granulation step.

Said pharmaceutical composition may be used as a medicament, particularly in the treatment of chronic lymphocytic leukaemia (CLL), mantle cell lymphoma (MCL) and Waldenström's macroglobulinaemia (WM).

### BRIEF DESCRIPTION OF THE DRAWINGS

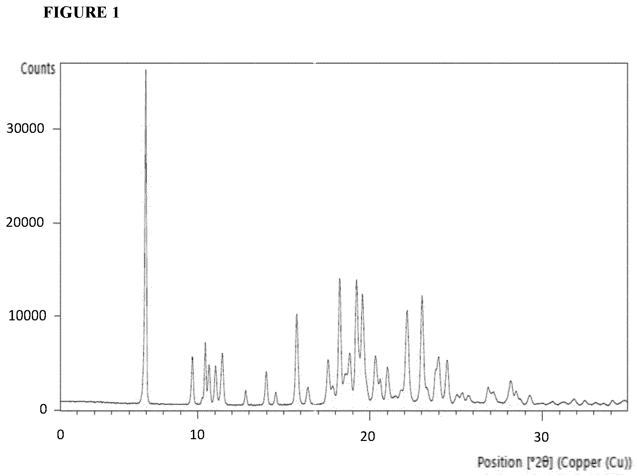

Figure 1 shows the full XRPD pattern of ibrutinib form C. For measurement conditions see the Examples section.

### DETAILED DESCRIPTION OF THE PRESENT INVENTION

Imbruvica^{®} contains the crystalline ibrutinib form A as disclosed in WO2013184572. Ibrutinib from A is a very stable compound, but being a BCS class II compound, it exhibits low aqueous solubility which affects dissolution behavior. The compound is slightly soluble at pH 1.2 and practically insoluble in the pH range from 3 to 8.

Other forms of ibrutinib have been disclosed in the prior art. Some of these forms are solvated forms of ibrutinib and some of them do contain unwanted (e.g. toxic) solvents. Moreover, some of the forms as disclosed in the prior art are rather unstable.

WO2013184572 discloses, besides ibrutinib form A, crystalline forms B and C and solvated forms D (methyl isobutyl ketone solvate), E (toluene solvate) and F (methanol solvate). The XRPD pattern of ibrutinib form C is shown in figure 1. This crystalline form is less stable than ibrutinib form A, but exhibits a significantly higher aqueous solubility.

Both capsule and tablet composition of Imbruvica^{®} contain a considerable amount of the anionic surfactant sodium lauryl sulphate (SLS). It is known that this compound, like some other surfactants, may give rise to irritation of the gastrointestinal tract. It would thus be advantageous to have a pharmaceutical composition comprising ibrutinib that does not contain any surfactant.

It was surprisingly found by the present inventors that a tablet composition exhibiting a similar dissolution profile as Imbruvia^{®} and which is completely free of surfactant, can be obtained by using ibrutinib form C. The tablet composition of the present invention is very stable and even after storage at elevated temperature or increased relative humidity, ibrutinib form C in the composition does not convert into ibrutinib form A or any other crystalline form of ibrutinib. The tablet is prepared by a robust and cost effective process and is bioequivalent to Imbruvica^{®}.

The present invention thus provides a tablet composition comprising ibrutinib and one or more pharmaceutically acceptable excipients, characterized in that:
* The one or more pharmaceutically acceptable excipients are 10 to 30% w/w of one or more diluents, 0 to 7% w/w of one or more binders, 2 to 15% w/w of one or more disintegrants, 0.25 to 1.0% w/w of one or more glidants and 0.25 to 2.0% w/w of one or more lubricants; and
- Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation; and
- Ibrutinib form C is present in an amount from 60 to 80% w/w relative to the total weight of the tablet; and
- The composition is free of amiphiphilic molecules that contain both hydrophilic and lipophilic groups and have HLB (hydrophile-lipophile balance) number above 10; and
- The composition exhibits a dissolution rate of at least 65% in 20 minutes when tested in 900 ml 0.01 N hydrochloric acid pH 2.0 (+ 1 % Tween 20) at 37°C, 75 rpm and/or in 900 ml phosphate buffer pH 6.8 (+3% Tween 20) at 37°C, 75 rpm in a USP apparatus II.

The XRPD pattern of ibrutinib form C may further comprise characteristic peaks at the following 2 theta (± 0.2) angles: 15.7°, 17.5°, 20.3°, 22.1° and 24.0°, measured using a Cu Kα radiation. The XRPD pattern of ibrutinib form C is shown in figure 1.

The tablet composition in accordance with the present invention is free of surfactant. Surfactants are amphiphilic molecules that contain both hydrophilic and lipophilic groups. Surfactants may have several uses in pharmaceuticals, e.g. to solubilize hydrophobic drugs in aqueous media or to improve drug absorption and penetration. Surfactants are classified into ionic surfactants and non-ionic surfactants. Ionic surfactants are subclassified into anionic surfactants where the hydrophilic group dissociates into anions in aqueous solutions, cationic surfactants that dissociate into cations and amphoteric surfactants that dissociate in anions and cations often depending on pH. Examples of non-ionic surfactants are polyol esters including glycol and glycerol esters and sorbitan derivatives, polyoxyethylene esters including polyethylene glycol and poloxamers, which are non-ionic triblock copolymers composed of polyoxypropylene flanked by polyoxyethylene. Examples of ionic surfactants are sodium lauryl sulfate, docusate, alkyl ether phosphates and quartenary ammonium salts. The hydrophile-lipophile balance (HLB) number is used as a measure of the ratio of the hydrophilic and lipophilic groups in the surfactant and defines the affinity for water or oil. HLB numbers above 10 indicate an affinity for water (hydrophilic) and below 10 an affinity for oil (lipophilic).

The tablet composition of the present composition is free of amiphiphilic molecules that contain both hydrophilic and lipophilic groups and have HLB (hydrophile-lipophile balance) number above 10, **and does thus** not contain SLS or any other surfactant. It was found that the presence of surfactant in the tablet composition triggers conversion of ibrutinib form C into form A in the composition of the present invention. Especially hydrophilic surfactants give rise to polymorphic conversion. Main advantage of not having surfactant in the tablet composition is that irritation, of *e.g.* gastrointestinal tract, is prevented.

Ibrutinib form C is present in the tablet composition of the present invention in an amount from 60 to 80% w/w relative to the total weight of the tablet. More preferably, ibrutinib form C is present in the tablet composition in an amount from 65 to 75% w/w relative to the total weight of the tablet.

The tablet compositions according to the present invention display dissolution behavior typical for immediate-release formulations. The compositions of the present invention exhibit a dissolution rate of at least 65% in 20 minutes when tested in 900 ml 0.01 N hydrochloric acid pH 2.0 (+ 1 % Tween 20) at 37°C, 75 rpm and/or in 900 ml phosphate buffer pH 6.8 (+3% Tween 20) at 37°C, 75 rpm in a USP apparatus II.

The tablet compositions according to the present invention comprise, besides ibrutinib form C, one or more pharmaceutically acceptable excipients. The excipients to be used in accordance with the present invention are well-known and are those excipients which are conventionally used by the person skilled in the art. The pharmaceutically acceptable excipients are chosen from one or more diluents, binders, disintegrants, glidants or lubricants.

The pharmaceutical composition according to the present invention comprises preferably 10-30% w/w of one or more diluents, 0-7% w/w of one or more binders, 2-15% w/w of one or more disintegrants, 0.25-1.0% w/w of one or more glidants and 0.25-2.0% w/w of one or more lubricants, all relative to the total tablet weight.

The diluent to be used in accordance with the present invention may be any diluent known to a person of ordinary skill in the art. Particularly, the diluent to be used in accordance with the present invention is an inorganic diluent, polysaccharide, mono- or disaccharide or sugar alcohol. Lactose and microcrystalline cellulose are particularly preferred diluents.

In one embodiment of the present invention, the diluent is added as intragranular component. In another embodiment, the diluent is added partially to the intragranular phase and partially to the extragranular phase.

In another embodiment of the present invention, the diluent to be used in accordance with the present invention is a basifying excipient, e.g. metal carbonate or bicarbonate. This type of diluent may decrease the dissolution rate of the tablet comprising ibrutinib by way of its basic character. A particularly preferred basifying excipient is sodium bicarbonate.

The diluent to be used in accordance with the present invention may contain lactose, microcrystalline cellulose, a basifying excipient or mixtures thereof.

The binder to be used in accordance with the present invention may be any binder known to a person of ordinary skill in the art. Suitable binders are selected from the group consisting of sodium carboxymethylcellulose, polyvinyl pyrrolidone (PVP), copovidone, polyvinyl pyrrolidone-vinyl acetate (PVP/VA) copolymer, hydroxypropylcellulose (HPC), hydroxypropyl methylcellulose (HPMC) or ethyl cellulose. PVP, copovidone and HPMC are particularly preferred binders.

The binder is added to the intragranular phase.

The disintegrant to be used in accordance with the present invention may be any disintegrant known to a person of ordinary skill in the art. Suitable disintegrants to be used in accordance with the present invention are selected from the group consisting of croscarmellose sodium, crospovidone, low-substituted hydroxypropylcellulose (HPC) or sodium starch glycolate. Croscarmellose sodium is a particularly preferred disintegrant.

In one embodiment of the present invention, the disintegrant is added as to the intragranular phase. In another embodiment, the disintegrant is added partially to the intragranular phase and partially to the extragranular phase.

The glidant to be used in accordance with the present invention may be any glidant known to a person of ordinary skill in the art. Colloidal silicon dioxide is a particularly preferred glidant.

In one embodiment of the present invention, the glidant is added as intragranular component. In another embodiment, the glidant is added to the extragranular phase.

The lubricant to be used in accordance with the present invention may be any lubricant known to a person of ordinary skill in the art. Magnesium stearate is a particularly preferred lubricant.

The lubricant may be added as extragranular component or it may be divided over the intragranular and extragranular phase. In a preferred embodiment, the lubricant is added partially to the intragranular phase and partially to the extragranular phase.

The tablets may be optionally further coated by a film-coat. The coating serves generally for cosmetic purposes. The coating material typically has no influence on the release rate, except of an inherent short initial delay in dissolution due to the time necessary to dissolve the coat. The coating may be selected from amongst one or more of those suitable coating materials known in the art.

The coating may be performed by applying one or more film forming polymers, with or without other pharmaceutically inert excipients, as a solution/suspension. Coating is done using any conventional coating technique known in the art, such as spray coating in a conventional coating pan or fluidized bed processor; or dip coating.

The tablet composition according to the present invention is packaged in primary packaging material, e.g. blisters and bottles. The tablet composition of the present invention is preferably packaged in capped bottles. HDPE bottles are particularly preferred. The capped bottles may comprise means to absorb water by having a cap containing desiccant, e.g. silica gel.

The pharmaceutical composition of the present invention exhibits excellent long term stability. After storage of the composition for 3 months at 40°C/75% RH, XRPD analysis showed that ibrutinib was kept in form C and did not convert into any other polymorphic form. Moreover, the pharmaceutical composition of the present invention is very suitable for production on commercial scale making use of equipment and techniques commonly used in industry.

The present invention further provides a process to prepare a tablet composition comprising ibrutinib form C and one or more pharmaceutically acceptable excipients comprising a granulation step. The granulation processes applied are simple and cost effective and include a standard wet or dry granulation technique.

The wet granulation process is performed with a granulation solvent selected from the group consisting of water, acetone, ethanol, isopropanol or a mixture thereof.

Preferably, the process to prepare the tablet composition of the present invention comprises a dry granulation step. The dry granulation process is conducted by either slugging or roller compaction. The advantage of the dry granulation over the process of wet granulation is that it does not use any organic solvents or water. The risk of stability issues is minimized in this way, especially when active pharmaceutical ingredients are used that are prone to (polymorphic) conversion. However, due to the relative high amount of API in the tablet composition of the present invention, applying the process of dry granulation is not the obvious choice.

The tablet prepared by applying the step of dry granulation comprises, besides ibrutinib form C, one or more pharmaceutically acceptable binders, diluents, disintegrants, glidants or lubricants. Preferably, the tablet prepared by using the step of dry granulation comprises ibrutinib form C, lactose, microcrystalline cellulose, polyvinylpyrrolidone (PVP), croscarmellose sodium, silicon dioxide and magnesium stearate. Optionally, HPMC may be present as well.

The tablet composition in accordance with the present invention may be used as a medicament. The composition typically may be used in the treatment of chronic lymphocytic leukaemia (CLL), mantle cell lymphoma (MCL) and Waldenström's macroglobulinaemia (WM).

The following examples are intended to illustrate the scope of the present invention but not to limit it thereto.

### EXAMPLES

The full XRPD pattern of ibrutinib form C of Figure 1 was obtained using a Bruker-AXS D8 Vario diffractometer with θ/2θ geometry (reflection mode), equipped with a Lynxeye detector and applying the following measurement conditions:
Start angle (2θ): 2.0°
End angle (2θ): 35.0°
Scan step width: 0.02°
Scan step time: between 0.2-2.0 seconds
Radiation type: Cu
Radiation wavelengths: 1.5406Å (Kα1), primary monochromator used
Exit slit: 6.0 mm
Focus slit: 0.2 mm
Divergence slit: Variable (V20)
Antiscatter slit: 11.8 mm
Receiving slit: 20.7 mm

### Example 1

The film-coated tablets comprising ibrutinib form C have the composition as given in table 1.

**Table 1**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose anhydrous | 112.0 | 14.0 |
| PVP K-25 | 16.0 | 2.0 |
| Croscarmellose sodium | 40.0 | 5.0 |
| Magnesium stearate | 4.0 | 0.5 |

| *Extragranular components* | | |
|---|---|---|
| Microcrystalline cellulose | 44.0 | 5.5 |
| Croscarmellose sodium | 16.0 | 2.0 |
| Colloidal silicon dioxide | 4.0 | 0.5 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 2

The film-coated tablets comprising ibrutinib form C were prepared by the process of dry granulation and have the composition as given in table 2.

**Table 2**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 112.0 | 14.0 |
| PVP K-25 | 16.0 | 2.0 |
| Croscarmellose sodium | 40.0 | 5.0 |
| Colloidal silicon dioxide | 4.0 | 0.5 |
| Microcrystalline cellulose | 44.0 | 5.5 |
| Magnesium stearate | 4.0 | 0.5 |

| *Extragranular components* | | |
|---|---|---|
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 3

The film-coated tablets comprising ibrutinib form C were prepared by the process of dry granulation and have the composition as given in table 3.

**Table 3**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 112.0 | 14.0 |
| PVP K-25 | 16.0 | 2.0 |
| Croscarmellose sodium | 40.0 | 5.0 |
| Colloidal silicon dioxide | 4.0 | 0.5 |
| Magnesium stearate | 4.0 | 0.5 |

| *Extragranular components* | | |
|---|---|---|
| Microcrystalline cellulose | 44.0 | 5.5 |
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 4

The film-coated tablets comprising ibrutinib form C were prepared by the process of dry granulation and have the composition as given in table 4.

**Table 4**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 112.0 | 14.0 |
| PVP K-25 | 16.0 | 2.0 |
| Croscarmellose sodium | 40.0 | 5.0 |
| Colloidal silicon dioxide | 4.0 | 0.5 |
| Microcrystalline cellulose | 44.0 | 5.5 |
| Magnesium stearate | 4.0 | 0.5 |

| *Extragranular components* | | |
|---|---|---|
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 5

The film-coated tablets comprising ibrutinib form C were prepared by the process of dry granulation and have the composition as given in table 5.

**Table 5**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 142.0 | 17.75 |
| PVP K-25 | 16.0 | 2.0 |
| Croscarmellose sodium | 10.0 | 1.25 |
| Colloidal silicon dioxide | 4.0 | 0.5 |
| Magnesium stearate | 4.0 | 0.5 |

| *Extragranular components* | | |
|---|---|---|
| Microcrystalline cellulose | 44.0 | 5.5 |
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 6

The film-coated tablets comprising ibrutinib form C were prepared by the process of dry granulation and have the composition as given in table 6.

**Table 6**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 112.0 | 14.0 |
| PVP K-25 | 16.0 | 2.0 |
| HPMC | 40.0 | 5.0 |
| Colloidal silicon dioxide | 4.0 | 0.5 |
| Magnesium stearate | 4.0 | 0.5 |

| *Extragranular components* | | |
|---|---|---|
| Microcrystalline cellulose | 44.0 | 5.5 |
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches.

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

### Example 7:

The film-coated tablets comprising ibrutinib form C were prepared by the process of dry granulation and have the composition as given in table 7.

**Table 7**

| | **Ibrutinib tablet composition** | |
|---|---|---|
| **Component** | **mg/tablet** | **%** |
| *Intragranular components* | | |
| Ibrutinib form C | 560.0 | 70.0 |
| Lactose monohydrate | 128.0 | 16.0 |
| HPMC | 40.0 | 5.0 |
| Colloidal silicon dioxide | 4.0 | 0.5 |
| Magnesium stearate | 4.0 | 0.5 |

| *Extragranular components* | | |
|---|---|---|
| Microcrystalline cellulose | 44.0 | 5.5 |
| Croscarmellose sodium | 16.0 | 2.0 |
| Magnesium stearate | 4.0 | 0.5 |
| **Total core tablet weight** | **800.0** | **100.0** |

The tablets were prepared by using the process of dry granulation. The granulate was mixed with the extragranular components and compressed using a rotating tablet press using appropriate punches

The tablets were packed in Alu/Alu blisters and in HDPE bottles, comprising desiccant in the cap.

XRPD analysis performed after storing the tablets for 3 months at 40°C/75% RH showed that ibrutinib is present in form C; no conversion into any other polymorphic form of ibrutinib was observed.

The tablets obtained, exhibited a dissolution rate of at least 65% in 20 minutes when tested in 900 ml 0.01 N hydrochloric acid pH 2.0 (+ 1 % Tween 20) at 37°C, 75 rpm or in 900 ml phosphate buffer pH 6.8 (+3% Tween 20) at 37°C, 75 rpm in a USP apparatus II. The dissolution profiles of the tablets match the profiles of Imbruvica^{®}.

## Claims

1. A tablet composition comprising 60 to 80% w/w of ibrutinib, 10 to 30% w/w of one or more diluents, 0 to 7% w/w of one or more binders, 2 to 15% w/w of one or more disintegrants, 0.25 to 1.0% w/w of one or more glidants and 0.25 to 2.0% w/w of one or more lubricants , all relative to the total tablet weight, **characterized in that**:
• Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation; and
• The composition is free of amiphiphilic molecules that contain both hydrophilic and lipophilic groups and have HLB (hydrophile-lipophile balance) number above 10; and
• The composition exhibits a dissolution rate of at least 65% in 20 minutes when tested in 900 ml 0.01 N hydrochloric acid pH 2.0 (+ 1 % Tween 20) at 37°C, 75 rpm and/or in 900 ml phosphate buffer pH 6.8 (+3% Tween 20) at 37°C, 75 rpm in a USP apparatus II.

2. The tablet composition according to claim 1, wherein the diluent contains lactose, microcrystalline cellulose, a basifying excipient or mixtures thereof.

3. A process for preparing the tablet composition according to any one of claims 1 or 2 comprising a granulation step.

4. The process according to claim 3, wherein the step of granulation is a dry granulation step.

5. The tablet composition according to any one of claims 1 to 2 for use as a medicament.

6. The composition according to claim 5 for use in the treatment of chronic lymphocytic leukaemia (CLL), mantle cell lymphoma (MCL) and Waldenström's macroglobulinaemia (WM).

## Patentansprüche

1. Tablettenzusammensetzung, die 60 bis 80% Gew./Gew. Ibrutinib, 10 bis 30% Gew./Gew. eines oder mehrerer Verdünnungsmittel, 0 bis 7% Gew./Gew. eines oder mehrerer Bindemittel, 2 bis 15% Gew./Gew. eines oder mehrerer Sprengmittel, 0,25 bis 1,0% Gew./Gew. eines oder mehrerer Gleitmittel, und 0,25 bis 2,0% Gew./Gew. eines oder mehrerer Schmiermittel umfasst, alles relativ zum Tablettengesamtgewicht, **dadurch gekennzeichnet, dass**:
• Ibrutinib in Form C ist, mit charakteristischen Signalspitzen im Röntgenpulverdiffraktionsmuster bei den folgenden 2-Theta (± 0,2)-Winkeln: 6,9°, 18,2°, 19,2°, 19,6° und 23,0°, gemessen unter Verwendung von Cu-Kα-Strahlung; und
• die Zusammensetzung frei von amphiphilen Molekülen ist, die sowohl hydrophile als auch lipophile Gruppen enthalten und eine HLB-Zahl (Hydrophil-Lipophil-Balance) von über 10 aufweisen; und
• die Zusammensetzung eine Auflösungsrate von mindestens 65% in 20 Minuten aufweist, wenn sie in 900 ml 0,01 N Salzsäure pH 2,0 (+1% Tween 20) bei 37°C, 75 U/min, und/oder in 900 ml Phosphatpuffer pH 6,8 (+3% Tween 20) bei 37°C und 75 U/min in einem USP-Gerät II getestet wird.

2. Tablettenzusammensetzung nach Anspruch 1, wobei das Verdünnungsmittel Lactose, mikrokristalline Cellulose, einen basifizierenden Hilfsstoff oder Gemische davon enthält.

3. Verfahren zum Herstellen der Tablettenzusammensetzung gemäß einem beliebigen der Ansprüche 1 oder 2, umfassend einen Granulationsschritt.

4. Verfahren nach Anspruch 3, wobei der Schritt der Granulation ein Trockengranulationsschritt ist.

5. Tablettenzusammensetzung gemäß einem beliebigen der Ansprüche 1 bis 2 zur Verwendung als Arzneimittel.

6. Zusammensetzung gemäß Anspruch 5 zur Verwendung bei der Behandlung von chronischer lymphatischer Leukämie (CLL), Mantelzell-Lymphom (MCL) und Waldenström-Makroglobulinämie (WM).

## Revendications

1. Composition de comprimé comprenant 60 à 80 % p/p d'ibrutinib, 10 à 30 % p/p d'un ou plusieurs diluants, 0 à 7 % p/p d'un ou plusieurs liants, 2 à 15 % p/p d'un ou plusieurs agents de désagrégation, 0,25 à 1,0 % p/p d'un ou plusieurs agents de glissement et 0,25 à 2,0 % p/p d'un ou plusieurs lubrifiants, tous par rapport au poids total du comprimé, **caractérisée en ce que** :
• l'ibrutinib est la forme C, ayant des pics caractéristiques dans le diagramme de diffraction des rayons X sur poudre aux angles 2 thêta ± 0,2 suivants : 6,9°, 18,2°, 19,2°, 19,6° et 23,0°, mesurés à l'aide d'un rayonnement Cu Kα ; et
• la composition est exempte de molécules amphiphiles qui contiennent à la fois des groupes hydrophiles et lipophiles et ont un nombre HLB (équilibre hydrophile-lipophile) supérieur à 10 ; et
• la composition présente une vitesse de dissolution d'au moins 65 % en 20 minutes lorsqu'elle est testée dans 900 ml d'acide chlorhydrique 0,01 N pH 2,0 (+ 1 % de Tween 20) à 37°C, 75 tr/min et/ou dans 900 ml de tampon phosphate pH 6,8 (+3 % de Tween 20) à 37 °C, 75 tr/min dans un appareil USP II.

2. La composition de comprimé selon la revendication l, dans laquelle le diluant contient du lactose, de la cellulose microcristalline, un excipient basifiant ou des mélanges de ceux-ci.

3. Procédé de préparation de la composition de comprimé selon l'une quelconque des revendications 1 ou 2, comprenant une étape de granulation.

4. Le procédé selon la revendication 3, dans lequel l'étape de granulation est une étape de granulation à sec.

5. La composition de comprimé selon l'une quelconque des revendications 1 à 2 pour une utilisation comme médicament.

6. La composition selon la revendication 5 pour une utilisation dans le traitement de la leucémie lymphoïde chronique (LLC), du lymphome à cellules du manteau (LCM) et de la macroglobulinémie de Waldenström (WM).
